(19) Europäisches Patentamt / European Patent Office / Office européen des brevets

(11) **EP 4 000 669 A1**

(12) **EUROPEAN PATENT APPLICATION**
published in accordance with Art. 153(4) EPC

(43) Date of publication:
**25.05.2022  Bulletin 2022/21**

(21) Application number: **19937828.2**

(22) Date of filing: **15.07.2019**

(51) International Patent Classification (IPC):
**A61M 5/172** *(2006.01)*

(52) Cooperative Patent Classification (CPC):
**A61M 5/172**

(86) International application number:
**PCT/CN2019/096009**

(87) International publication number:
**WO 2021/007749 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shenzhen Mindray Scientific Co., Ltd.**
**Shenzhen, Guangdong 518000 (CN)**

(72) Inventors:
• ZUO, Pengfei
  Shenzhen, Guangdong 518000 (CN)
• TU, Youqiang
  Shenzhen, Guangdong 518000 (CN)

(74) Representative: **KIPA AB**
**Drottninggatan 11**
**252 21 Helsingborg (SE)**

(54) **INFUSION STATE DETECTION METHOD FOR INFUSION PUMP, INFUSION PUMP, MEDICAL DEVICE AND STORAGE MEDIUM**

(57)     Disclosed are an infusion state detection method for an infusion pump (100), an infusion pump (100), a medical device (10) and a storage medium. The method comprises: acquiring a sensing signal fed back by a sensor arranged on an infusion apparatus (30) (S100); determining information of the liquid level state in the infusion apparatus (30) according to the sensing signal (S102); and according to the liquid level state information, triggering the execution of at least one of the following events: outputting alarm information, driving an extrusion mechanism (113) of an infusion pump (100) to stop moving, and driving the extrusion mechanism (113) of the infusion pump (100) to slow down so as to adjust the infusion flow rate of the infusion pump (100) (S104). The information of the liquid level state in the infusion apparatus (30) is determined by means of the sensing signal fed back by the sensor, and when the liquid level state information of the infusion apparatus (30) is determined, the warning information is output, or, the motion state of the extrusion mechanism (113) is controlled, such that extra operations of medical staff are reduced, thereby reducing the probability of medical accidents.

FIG.1

EP 4 000 669 A1

**Description**

<u>TECHNICAL FIELD</u>

The disclosure relates to the technical field of medical treatment, and more particularly to an infusion state detection method for an infusion pump, an infusion pump, a medical device and a storage medium.

<u>BACKGROUND</u>

The infusion pump infuses by squeezing an infusion apparatus via a finger-shaped pump sheet which is periodically peristaltic. With the passage of time, the drugs in the infusion bag are gradually input into the body of the patient. When the infusion of drugs in the infusion bag is completed, reminding the medical staff to replace the infusion bag timely can prevent the delay of the treatment process, or reminding the medical staff to shut down the infusion pump timely can reduce the probability of medical accidents. Therefore, it is necessary to detect the infusion state.

<u>SUMMARY</u>

Embodiments of the present disclosure has disclosed a method and an electronic device for automatically determining a state information for liquid level in an infusion apparatus and performing a corresponding operation.

In a first aspect of an embodiment of the present disclosure, an infusion state detection method for an infusion pump which is applied to an infusion pump system, is provided, wherein the infusion pump system includes an infusion pump and an infusion apparatus which is arranged along an infusion pipeline; wherein the infusion state detection method for an infusion pump includes:

acquiring a sensed signal which is fed back by a sensor, wherein the sensor is arranged on the infusion apparatus;
determining state information of a liquid level in the infusion apparatus according to the sensed signal;
triggering implementation of at least one of following events according to the state information of the liquid level:

outputting alarm information,
driving an extrusion mechanism of the infusion pump to stop moving, or
driving the extrusion mechanism of the infusion pump to slow down so as to adjust an infusion flow rate of the infusion pump.
In a second aspect of an embodiment of the present disclosure, an infusion pump is provided, wherein the infusion pump is used together with an infusion apparatus and is operable to implement an infusion and injection operation on a liquid which is prepared by a user; wherein the infusion pump includes a processor, an output interface, a sensor, a drive mechanism and an extrusion mechanism; wherein the processor is operable to drive the drive mechanism to enable the extrusion mechanism to squeeze the liquid in the infusion apparatus for moving the liquid according to a preset direction;
the infusion apparatus is arranged along an infusion pipeline, the sensor is arranged on the infusion apparatus;
wherein the sensor is operable to feed back to the processor a sensed signal which is associated with a state of the infusion apparatus;
the processor is further operable to acquire the sensed signal which is fed back by the sensor, to determine state information of a liquid level in the infusion apparatus according to the sensed signal, and to trigger implementation of at least one of following events according to the state information of the liquid level:

outputting alarm information through the output interface,
driving the extrusion mechanism of the infusion pump to stop moving, or
driving the extrusion mechanism of the infusion pump to slow down so as to adjust a flow rate of the infusion pump.

In a third aspect of an embodiment of the present disclosure, an infusion pump is provided, wherein the infusion pump is used together with an infusion apparatus and is operable to implement an infusion and injection operation on a liquid which is prepared by a user; wherein the infusion pump includes a processor, an output interface, a sensor, a drive mechanism, an extrusion mechanism, a position sensor and a liquid stopping clip, wherein the infusion apparatus is arranged along an infusion pipeline and the sensor is arranged on the infusion pipeline;

wherein the sensor is operable to feed back to the processor a sensed signal which is associated with a state of the infusion apparatus, the position sensor is operable to detect an arrangement state of the infusion apparatus on the infusion pipeline and to send the arrangement state of the infusion apparatus to the processor;
wherein the processor is operable to drive the drive mechanism to enable the extrusion mechanism to squeeze the liquid in the infusion apparatus for moving the liquid according to a preset direction, to acquire the sensed signal which is fed back by the sensor, to determine state information of a liquid level in the infusion apparatus according to the sensed signal, and to trigger implementation of at least one of following events according to the state information of the liquid level:

outputting alarm information through the output interface,
driving the extrusion mechanism of the infusion pump to stop moving, or
driving the extrusion mechanism of the infusion pump to slow down so as to adjust a flow rate of the infusion pump;
wherein the processor is further operable to drive the liquid stopping clip to open, when determining that at least a part of the infusion apparatus is separated from the infusion pipeline according to the arrangement state of the infusion apparatus after the extrusion mechanism of the infusion pump stops moving.

In a fourth aspect of an embodiment of the present disclosure, a medical device is provided, wherein the medical device is connected with the infusion pump according to the second or third aspect discussed above in a detachable fixation manner, wherein an input interface of the medical device is connected with the output interface, and a display device is arranged on the medical device to display the alarm information outputted by the output interface.

In a fifth aspect of an embodiment of the present disclosure, a computer-readable storage medium on which executable instructions are stored, is provided, wherein when the executable instructions are executed by a processor, some or all steps mentioned in the first aspect of an embodiment of the present disclosure can be implemented.

The embodiments of this disclosure determine the state information of the liquid level in the infusion apparatus according to the sensed signal which is fed back by the sensor, and output the alarm information or control the movement state of the extrusion mechanism when the state information of the liquid level in the infusion apparatus is determined. In this way, when the liquid in the infusion bag enters into the infusion apparatus, an instruction can be sent timely, or the movement of the extrusion mechanism can be controlled timely. This is beneficial to reduce the additional operation of the medical staff and reduce the probability of medical accidents.

BRIEF DESCRIPTION OF THE DRAWINGS

In order to explain embodiments of this disclosure more clearly, the following will briefly introduce drawings required in the description for the embodiments or the prior art description. It is obvious that the drawings in the following description are only some embodiments of this disclosure. For those skilled in the art, other drawings can be obtained from these accompanying drawings without paying any creative works.

FIG. 1 is a step flow diagram of an infusion state detection method for an infusion pump in an embodiment of the present disclosure.
FIG. 2 is a hardware structure block diagram of an infusion pump system in an embodiment of the present disclosure.
FIG. 3 is a hardware structure block diagram of a medical device in an embodiment of the present disclosure.
FIG. 4 is a schematic diagram showing a connection between a peristaltic extrusion mechanism and an infusion apparatus in an embodiment of the disclosure.
FIG. 5 is a schematic diagram showing a relationship between a pressure signal transmitted by a pressure sensor during infusion and an infusion time, in an embodiment of the present disclosure.
FIG. 6 is a schematic diagram showing a trend of change in pressure on a pipe wall under different modes, in an embodiment of the present disclosure.
FIG. 7 is a schematic diagram showing a connection between a medical device and an infusion pump, in an embodiment of the present disclosure.

DESCRIPTION OF THE PREFERRED EMBODIMENTS

The technical solutions in example embodiments of the disclosure will be described clearly and completely below with reference to the accompanying drawings. Apparently, the embodiments described are merely some, rather than all, of the embodiments of the disclosure. It should be understood that the disclosure is not limited by the example embodiments described herein. All other embodiments derived by those skilled in the art without creative efforts on the basis of the embodiments described in the disclosure shall fall within the scope of protection of the disclosure.

The expressions of "first" and "second" recited in the description, claims and accompanying drawings of this disclosure, are only to distinguish different objects, and do not intend to describe the specific sequence. In additional, the terms "comprising" and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these procedures, methods, or devices.

The expression "embodiment" when referred in the present disclosure, it means that the features, structures or characteristics described in connection with the embodiment may be included in at least one embodiment of the present disclosure. The phrase appearing in each position in this description does not necessarily refer to the same embodiment, nor refer to an independent or alternative embodiment mutually exclusive with other embodiments. Those skilled in the art explicitly and implicitly understand that the embodiments described herein can be combined with other embodiments. As used herein, depending on the context, the term "if" can be interpreted as meaning "when", "in response to determining" or "in response to detecting". Similarly, depending on the context, the phrase "if determined..." or "if [a stated condition or event] is detected" may be interpreted to mean "when determining...", "in response to determining...", "when [a stated condition or event] is detected" or "in response to a detection of [a stated condition or event]".

It should be noted that the following method embodiments are expressed as a series of action combinations for simple description. However, those skilled in the art should be aware that the disclosure is not limited by the action sequence described herein, because according to the disclosure, some steps can be carried out in other sequences or at the same time.

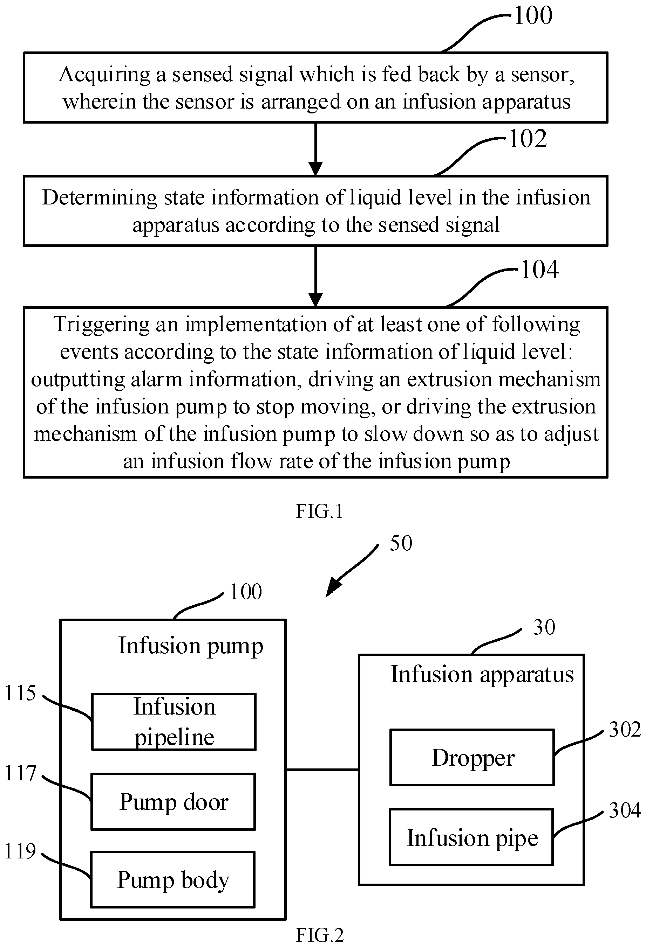

Referring FIG.1, a step flow diagram of an infusion state detection method for an infusion pump in an embodiment of the present disclosure is shown, which includes following steps.

In step S100, a sensed signal which is fed back by a sensor, is acquired. Wherein the sensor is arranged on an infusion apparatus.

Now refer FIG.2 simultaneously, which is a hardware structure block diagram of an infusion pump system in an embodiment of the present disclosure. The infusion pump system 50 includes an infusion pump 100 and an infusion apparatus 30. The infusion pump 100 includes a pump body 119 and a pump door 117 which is rotatably arranged on the pump body 119. The pump body 119 includes an infusion pipeline 115 which is arranged inside and the infusion apparatus 30 can be arranged along the infusion pipeline 115. The infusion apparatus 30 includes a dropper 302 and an infusion pipe 304, and the dropper 302 can be arranged on the infusion pipe 304.

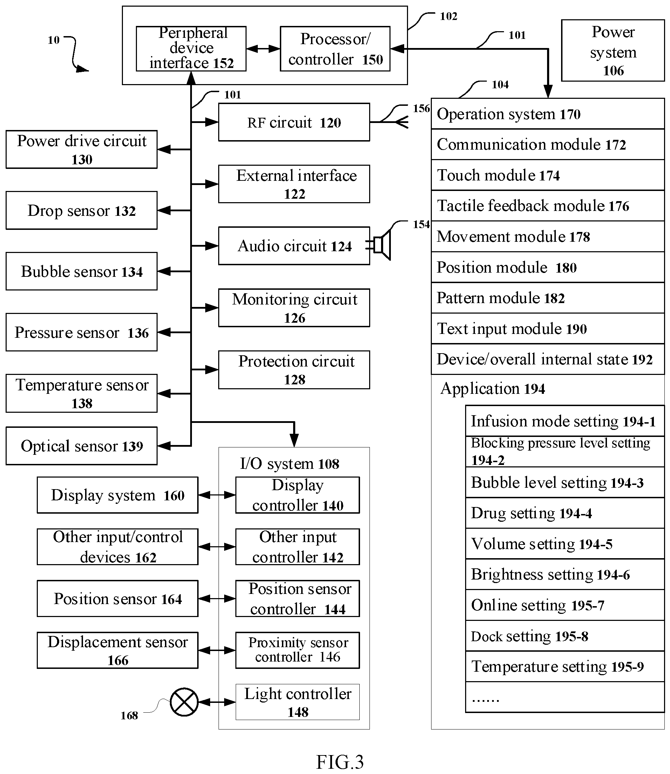

Now refer FIG.3 simultaneously, which is a hardware structure block diagram of a medical device in an embodiment of the present disclosure. The medical device 10 includes a control platform 102, a memory 104, a power supply system 106, an input/output (I/O) system 108, an RF circuit 120, an external interface 122, an audio circuit 124, a monitoring circuit 126, a protection circuit 128, a power drive circuit 130, a drop sensor 132, a bubble sensor 134, a pressure sensor 136, a temperature sensor 138 and so on. These components communicate with each other through one or more communication buses or signal lines 101. The control platform 102 includes a processor 150 and a peripheral device interface 152.

The medical device 10 may be any medical device that is capable of implementing infusion and injection operations which are preset by a user on liquid which is prepared by the user, for infusing or injecting the prepared drug liquid into a patient controllably. The medical device includes but is not limited to the infusion pump 100. In some embodiments, the medical device can be used together with the infusion apparatus. It should be understood that the medical device 10 is only an example, and the medical device may have more or fewer components than those are shown or have different component configurations. The various components described in FIG. 3 may be implemented in a hardware, a software, or a combination of software and hardware, and they can include one or more signal processing circuits and/or application specific integrated circuits.

The memory 102 may include a high-speed random-access memory and may also include a nonvolatile memory, such as one or more disk storage devices, flash memory devices, or other nonvolatile solid-state storage devices. In some embodiments, the memory 104 may also include a memory which is remote from one or more processers/controllers 150, such as an additional network memory accessed via the RF circuit 120 or via the external interface 122 and communication network (not shown). The communication network may be an Internet, one or more internal networks, a local area network (LAN), a wide area network (WLAN), a storage area network (SAN), etc., or an appropriate combination thereof. The processor/controller 150 may control access to the memory 104 by other components of the medical device 10 other than the peripheral device interface 152.

The peripheral device interface 152 couples input and output peripherals of the medical device 10 to the processor/controller 150 and the memory 104. For example, the peripheral device interface 152 may include an input interface and an output interface. The one or more processor/controllers 150 run various software programs and/or instruction groups stored in the memory 104 to perform various functions of the medical device 10 and process data.

In some embodiments, the peripheral device interface 152 and the processor/controller 150 may be implemented on a single chip. In some embodiments, they can be implemented on multiple discrete chips.

The RF (radio frequency) circuit 120 receives and transmits electromagnetic waves. The RF circuit 120 converts electrical signals into electromagnetic waves, or converts electromagnetic waves into electrical signals, and communicates with communication networks and other communication devices via electromagnetic waves. The RF circuit 120 may include well-known circuits for performing these functions, and these well-known circuits include but are not limited to the antenna system 156, a RF transceiver, one or more amplifiers, a tuner, one or more oscillators, a digital signal processor, a CODEC chipset, a user identity module (SIM) card, a memory, etc. The RF circuit 120 may communicate with networks and other devices through a wireless communication, which may be the world wide web (WWW), an intranet and/or a wireless network such as a cellular telephone network, a wireless local area network (LAN) and/or a metropolitan area network (MAN). The wireless communication can use any of a variety of communication standards, protocols and technologies, which include but are not limited to the global system for mobile communications (GSM), enhanced data GSM environment (EDGE), wideband code division multiple access (WCDMA), code division multiple access (CDMA), time division multiple access (TDMA), Bluetooth (such as ieee802.15.1), wireless fidelity (WiFi) (e.g., IEEE802.11a, IEEE 802.11b, IEEE 802.11g and/or IEEE 802.11n), voice over internet protocol (VoIP), WI-MAX, protocols for e-mail, instant messaging and/or short message service (SMS), or any other suitable communication protocols which include those are not yet developed at the date of submission of this disclosure.

The external interface 122 provides a wired communication interface between the medical device 10, other devices (such as a Dock, central station, monitor, etc.) or users (computers or other communication devices). In some embodiments, it can be a communication interface controlled by a CAN bus protocol, a communication interface controlled by a serial communication protocol (such as RS485, RS232), or a universal serial bus (USB). The external interface 122 is suitable for being directly or indirectly coupled to other devices or users via a network (such as the Internet, LAN, etc.).

The audio circuit 124 and the speaker 154 provide an audio interface between the user and the medical device 10. The audio circuit 124 receives audio data outputted from the peripheral device interface 152 through the output interface, converts the audio data into an electrical signal, and transmits the electrical signal to the speaker 154. The speaker 154 converts the electrical signal into a sound wave that can be perceived by human.

The monitoring circuit 126 may include a fault detection circuit which is operable to instruct a state of one or more processors/controllers 150.

The protection circuit 128 may include a hardware protection device (e.g., fuses, TVS diodes) which is operable to protect the electrical safety of various components in the medical device 10. The processor/controller 150 drives the power device (such as the peristaltic extrusion mechanism 110) of the medical device 10 through the power drive circuit 130, so that the power device can be moved controllably under the drive of the processor/controller 150. In the process of movement, one or more force transmission/conversion devices (such as gears or transmission shafts) drive the controlled object (such as a pump door, liquid stopping clip or peristaltic extrusion mechanism) to move. The power device can be an electromagnetic device that realizes the electric energy conversion or transmission according to the law of electromagnetic induction, such as a permanent magnet (PM) motor, a reactive (VR) motor and a hybrid (HB) motor. In some embodiments, the motor is driven by the processor/controller 150 to enable the controlled object of the medical device 10 (such as the pump door 117, the liquid stopping clip or the pump vane 114) to move, such that a preset movement state of the controlled object can be realized.

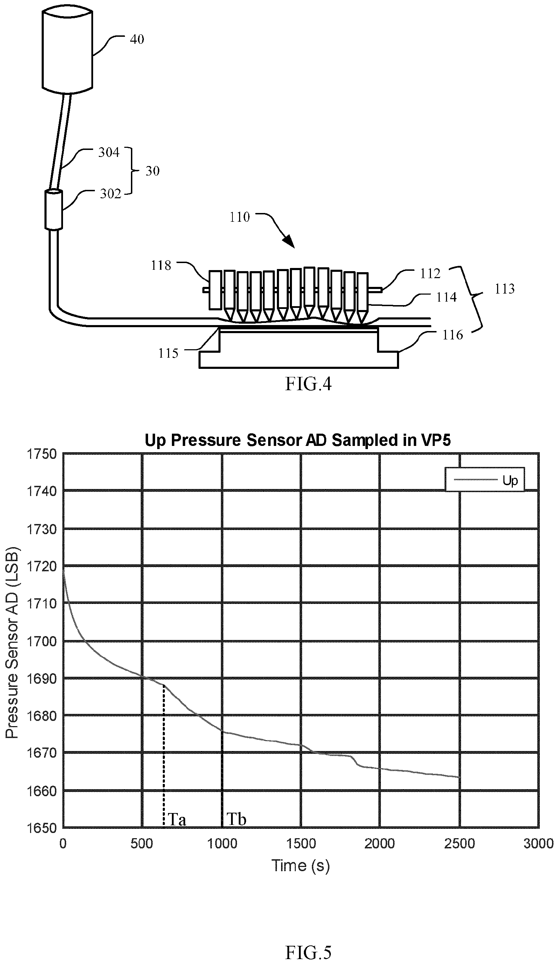

Now refer FIG.4 simultaneously, which is a schematic diagram showing a connection between a peristaltic extrusion mechanism and an infusion apparatus in an embodiment of the disclosure. In some embodiments, the peristaltic extrusion mechanism 110 includes a drive mechanism 118 and an extrusion mechanism 113, wherein the extrusion mechanism 113 includes a camshaft 112, a pump vane 114 and an extrusion plate 116. The processor/controller 150 in the medical device 10 sends a command, such as a speed command or a position command, drives the drive mechanism 118 (such as a motor) to operate according to a designated speed and direction through the power drive circuit 130. The drive mechanism 118 drives the camshaft 112 which is connected thereof to rotate during the rotation of drive mechanism 118. During the rotation of the camshaft 112, the pump vane 114 on camshaft 112 performs a linear reciprocating movement. That is, the pump vane performs the linear reciprocating movement in sequence. The pump vane 114 cooperates with the extrusion plate 116 to successively press and release the outer wall of the infusion apparatus 30, thus driving the liquid in the infusion apparatus 30 to flow continuously and directionally. A deceleration mechanism can also be arranged between the drive mechanism 118 and the camshaft 112 to ensure the smooth and uniform rotation speed of the pump vane 114. In this embodiment, the peristaltic extrusion mechanism 110 can include multiple pump vanes 114. The specific number of pump vanes 114 is not limited in this disclosure. The extrusion mechanism 113 can also include only a camshaft 112 and a pump vane 114. The extrusion plate 116 can be arranged inside the pump door 117. In this way, when the user closes the pump door 117, the extrusion plate 116 can contact the infusion pipe 304.

In some embodiments, the drop sensor 132 may be used in conjunction with the dropper 302 of the infusion apparatus 30 to detect a flow rate or flow quantity of liquid drops in the dropper 302.

In some embodiments, one or more bubble sensors 134 are operable to detect a presence and size of gas in the infusion apparatus 30. The bubble sensor 134 may be an ultrasonic sensor, an infrared sensor, or the likes.

In some embodiments, the pressure sensor 136 may respond to a pressure value on the measured object (such as the pipe wall of the infusion pipe 304), convert the pressure value into an electrical signal which is detectable and send it to the control platform 102. The pressure sensor 136 may be a resistance strain gauge pressure sensor, a semiconductor strain gauge pressure sensor, a piezoresistive pressure sensor, an inductive pressure sensor, a capacitive pressure sensor, a resonant pressure sensor, an optical fiber pressure sensor, or a capacitive acceleration sensor. In some embodiments, the pressure sensor 136 may be operable to detect an internal pressure of the infusion apparatus 30 or an external pressure of the infusion apparatus 30. In some embodiments, the pressure sensor 136 can also be operable to detect an in-position state of the measured object (such as the infusion pipe 304 or a syringe, etc.). In some embodiments, the pressure sensor 136 may detect a blockage in the infusion apparatus 30 or whether the infusion apparatus 30 leaks.

In some embodiments, the medical device 10 has a heating device for heating the liquid in the infusion apparatus 30. At this time, the temperature sensor 138 can be operable to detect the real-time temperature of the liquid. At the same time, the temperature value is converted into an electrical signal which is detectable and then sent to the control platform 102. The control platform 102 can display the real-time temperature through the display system 160 or control an on/off state of the heating device according to the temperature value.

In some embodiments, the optical sensor 139 may be arranged at a preset position of the infusion apparatus 30 to detect an information of liquid level in the infusion pipe 304 at the preset position. At the preset position, if the processor/controller 150 detects a first state information through the optical sensor 139, it indicates that there is liquid in the infusion pipe 304 at the preset position. That is, the liquid level in the infusion pipe 304 is not below the preset position. If the processor/controller 150 detects a second state information through the optical sensor 139, it indicates that the liquid level in the infusion pipe 304 at the preset position has fallen below the preset position. That is, gas has passed through the infusion pipe 304 at the preset position, and the liquid level in the infusion bag 40 has fallen to the preset position or below the preset position.

The input/output (I/O) system 108 provides an interface between the input/output peripherals of the medical device 10 and the peripheral device interface 152. The input/output peripherals may be a display system 160, a position sensor 164, a displacement sensor 166, a light component 168, and other input/control devices 162. The I/O system 108 may include a display controller 140, a position sensor controller 144, a proximity sensor controller 146, a light controller 148, and one or more other input controllers 142. One or more controllers in the I/O system 108 receive/transmit electrical signals from/to input/output peripherals. One or more input controllers 142 receive/transmit electrical signals from/to other input/control devices 162. The other input/control devices 162 may include a physical button (such as, a press button, a rocker button or a touch button, etc.), a slider switch, a joystick, etc. In some embodiments, the other input/control device 162 may include a physical button for emergency stop of infusion.

In some embodiments, the display system 160 may include a display screen that provides an output interface between the medical device 10 and the user. The display screen displays electronic documents on the screen through a specific transmission device and then reflects them to the human eye. The display screen can include a cathode ray pipe display (CRT), a plasma display (PDP) or a liquid crystal display (LCD), etc. In some embodiments, the display system 160 may include a touch screen that provides an input/output interface between the medical device 10 and the user. The touch screen can be an inductive display device that can receive input signals such as contacts, and include a resistance screen, a surface acoustic wave screen, an infrared touch screen, an optical touch screen, a capacitive screen or a nano film. Both of the display screen and touch screen can display the visual output to the user, such as through the output interface in the peripheral device interface 152. Visual output optionally includes pattern, text, chart, video, and combinations thereof. Some or all visual outputs can correspond to user interface objects, which will be described in more detail in this disclosure.

The touch screen also receives an input of user based on touch and/or contact. The touch screen forms a touch sensitive surface that receives the input of user. The touch screen and display controller 140 (together with any associated modules and/or instruction group in the memory 104) detects contact on the touch screen (and any movement or interruption of the touch) and converts the detected contact into an interaction with user interface objects such as one or more software keys displayed on the touch screen. In one exemplary embodiment, the contact point between the touch screen and the user corresponds to one or more fingers of the user. The touch screen can use LCD (liquid crystal display) technology or LPD (light emitting polymer display) technology. However, other display technologies can be used in other embodiments. The touch screen and display controller 140 may use any of a variety of touch sensitive technologies to detect the contact and its movement or interruption, which include but are not limited to capacitance, resistance, infrared and surface acoustic wave technologies, as well as other proximity sensor arrays, or other technologies for determining one or more points of contact with the touch screen.

The position sensor 164 can sense a position of the detected object, convert the position into an electrical signal which is detectable, and send the electrical signal to the control platform 102 through the I/O system 108. The position sensor 164 can be a contact sensor that generates a signal through contact and compression of two objects, such as a travel switch and a two-dimensional matrix position sensor; or can be a proximity sensor that generates a signal when two objects approach in a preset distance, such as an electromagnetic sensor, a photoelectric sensor, a differential transformer sensor, an eddy current sensor, a capacitive sensor, a reed switch sensor, an ultrasonic sensor or a hall sensor. The measured object can include the infusion apparatus, pump door, pump vane or liquid stopping clip, etc. In some embodiments, a hall position sensor may be operable to detect the position of the pump door. In some embodiments, a photoelectric position sensor may be operable to detect the position of the pump vane. In some embodiments, a photoelectric position sensor can be operable to detect whether the infusion apparatus is arranged at a preset position. In some embodiments, a photoelectric position sensor can be operable to detect the clipping position of the liquid stopping clip on the pipe.

In some embodiments, the control platform 102 can sense whether the infusion apparatus 30 is installed on the infusion line 115 through the position sensor 164. If the position sensor 164 detects that the pipe wall of the infusion apparatus 30 is not in contact with the infusion pipeline 115 within the detection range of the position sensor 164, the control platform 102 drives the liquid stopping clip to open and enable the liquid stopping clip to release the pipe wall of the infusion apparatus (such as the infusion pipe 304). Specifically, there can be more than two position sensors 164. When at least one position sensor 164 detects that the pipe wall of the infusion apparatus 30 is not in contact with the infusion pipeline 115, the control platform 102 can drive the liquid stopping clip to open. The infusion pipeline 115 refers to the place where the infusion apparatus is installed in the infusion pump.

The displacement sensor 166 can respond to a change of position of the measured object relative to a reference position, convert the change of position into an electrical signal which is detectable, and send the electrical signal to the control platform 102 through the I/O system 108. The displacement sensor 106 may be an inductive sensor, a capacitive sensor, an ultrasonic sensor or a hall sensor. In some embodiments, a potentiometer may be operable to monitor the change of position of the pump door.

The light component 168 may include a visual alarm element which indicates that the medical device 10 is in an abnormal state. Light component 168 individually responds to the drive of the processor/controller 150. The light component 168 may also cooperate with the speaker 154 to respond to the drive of the processor/controller 150. For example, the color or brightness of the light changes with the tone and frequency of the alarm sound. The light component 168 may include indicators for a power supply, a CPU and other components or an alarm indicator which indicates the infusion failure state. The light component 168 may also include a visual lighting element for facilitating the observation of the structure or component state of the medical device 10 when the ambient light is poor.

The medical device 10 also includes a power supply system 106 for supplying power to various components. The power system 106 may include a power management system, one or more power sources (e.g., batteries or alternating current (AC)), a charging system, a power failure detection circuit, a power converter or inverter, a power state indicator (e.g., light emitting diodes (LEDs)), or any other component associated with power generation, management and distribution.

In some embodiments, the software components include an operation system 170, a communication module (or instruction group) 172, a touch module (or instruction group) 174, a tactile feedback module (or instruction group) 176, a movement module (or instruction group) 178, a position module (or instruction group) 180, a pattern module (or instruction group) 182, a text input module (or instruction group) 190, a device/overall internal state (or instruction group) 192, and one or more applications (instruction group) 194.

Operation system 170 (e.g., embedded operation systems, such as Darwin, RTXC, LINUX, UNIX, OS, WINDOWS, etc.) includes various software components and/or drivers for controlling and managing conventional system tasks (e.g., memory management, storage device control, power management, etc.) and facilitating communication between various software and hardware components.

The communication module 172 facilitates to communicate with other devices via one or more external interfaces 122, and it also includes various software components which are operable to process data received by the RF circuit 120 and/or the external interface 122.

In some embodiments, the touch module 174 may selectively detect contact with the display system 160 or other touch sensitive devices (e.g., touch buttons, touch pads). For example, the touch module 174 detects contact with the display system 160 together with the display controller 140. The touch module 174 includes various software components which are operable to perform various operations associated with contact detection of the display system 160 (which can be detected by a finger or a stylus, etc.), such as operations of determining whether the contact occurs (e.g., detecting a press time of a finger), determining a strength of the contact (e.g., a force or pressure of the contact), determining whether the contact moves (e.g., detecting one or more finger dragging events), tracking the movement on the display, and determining whether the contact stops (e.g., detecting a lift time of the finger or a disconnection of the contact). Wherein the operation of determining a movement of a contact point may include determining a rate (amplitude), a speed (amplitude and direction), and/or an acceleration (including amplitude and/or direction) of the contact point. These operations can be applied to single-point contact or multi-point simultaneous contact. In some embodiments, the touch module 174 detects contact with other touch devices in conjunction with the display controller 140.

The touch module 174 may be used to detect gesture inputted by the user. Different gestures of the user on the touch sensitive device have different contact modes (for example, a detected contact position, contact time or contact intensity, or combinations of one or more thereof). For example, detecting gesture of tapping by a single finger includes detecting a finger press event, and then detecting a finger lift event at the same or similar position as the finger press event. For example, detecting gesture of swiping by a finger includes detecting a finger press event, and then monitoring one or more finger dragging events, and finally detecting a finger lift event. Similarly, the gestures of the touch pen, such as tapping, swiping, dragging and so on, are optionally detected by detecting a designated contact pattern of the touch pen.

The tactile feedback module 176 includes various software components which are operable to generate instructions for generating a tactile output at one or more positions of the medical device 10 by using one or more tactile output generators (not shown), in response to the interaction of the user with the medical device 10. For example, after detecting the contact on the surface of the touch device, the color of the pattern or text of the touch device changes, or a sound or vibration generates.

The position module 180 includes software components which are operable to perform various operations which are related to the detection of the device position and the detection of the change of the device position.

The pattern module 182 includes various known software components which are operable to render or display a pattern on the display screen of the display system 160 or other external devices, and includes components which are operable to change the visual impact (e.g., brightness, transparency, saturation, contrast or other visual attributes) of the displayed pattern. In the embodiments herein, the term "pattern" includes any object that can be displayed to the user, which includes but is not limited to text, web page, icon (e.g., user interface objects of soft keys), digital image, video, animation, and the likes. In some embodiments, the pattern module 182 stores data which represents the pattern to be used. Each pattern can be assigned a corresponding code. The pattern module 182 receives one or more codes for the pattern which is designated to be displayed from an application or the like, and also receives coordinate data together with other pattern attribute data if necessary, and then generates screen image data for outputting to the display controller 140.

The text input module 190 provides various software components which are operable to input text into one or more applications. Specifically, it can be used to input various infusion parameters, which include a drug name, infusion speed or alarm threshold.

In some embodiments, the memory 104 stores the device/overall internal state 192. The device/overall internal state 192 includes one or more of the followings: an active application state that indicates which applications (if any) are currently active; a display state that indicates what applications, views, or other information occupy various areas of the display system 160; a sensor state that includes information obtained from each sensor of the device and other input or control medical device 10; and position and/or orientation information about a position and/or posture of the device.

In some embodiments, the memory 104 stores at least one application 194, which may include an infusion mode setting 194-1, a blocking pressure level setting 194-2, a bubble level setting 194-3, a drug setting 194-4, a volume setting 194-5, a brightness setting 194-6, an online setting 195-7, a Dock setting 195-8, or a temperature setting 195-9. The infusion mode setting 194-1 can include a combination of preset infusion parameters for satisfying requirements of different use scenarios. The blocking pressure level setting 194-2 can include an interface for the user to input different blocking pressure levels. By inputting different blocking pressures, the blocking alarm threshold of the medical device 10 can be adjusted for satisfying requirements of different use scenarios. The bubble level setting 194-3 can include an interface for the user to input different bubble levels. By inputting different bubble levels, the bubble alarm threshold of the medical device 10 can be adjusted for satisfying requirements of different use scenarios. The drug setting 194-4 can include an interface for the user to input different drug names, drug abbreviations and/or drug colors. The drug parameters which are set before the infusion by inputting the corresponding drug names/abbreviations/colors, can facilitate the automatic confirmation inside the medical device 10 or the verification of medical staff during infusion. The volume setting 194-5 allows the user to adjust the volume of alarm and/or other audio output according to their requirements. The brightness setting 194-6 allows the user to adjust the brightness of the screen, alarm indicator, lighting lamp and so on, according to their requirements. The online setting 195-7 provides an input interface for the user to control the medical device 10 to work online with other devices or not and to control an online working mode according to their requirements. The Dock setting 195-8 provides a setting interface for the user to adjust the working parameters of the Dock connected to the medical device 10 according to their requirements. The temperature device 195-9 provides a setting interface for the user to heat the liquid in the infusion apparatus.

In this embodiment, the sensor can be arranged inside the infusion pump 100 or in a housing (not shown), and the housing is arranged independently of the infusion pump 100.

In one embodiment, the sensor may be one or more kinds of a pressure sensor or an optical sensor.

When the sensor is a pressure sensor, the pressure sensor can be arranged on the infusion pipe 304 between the dropper 302 and the extrusion mechanism 113 of the infusion pump 100 to detect pressure information on the pipe wall of the infusion apparatus 30 between the infusion apparatus 30 and the extrusion mechanism 113 of the infusion pump 100. In this embodiment, the pressure sensor feeds back multiple sensed signals to the infusion pump 100 so that the infusion pump 100 determines the pressure information on the pipe wall of the infusion apparatus 30 according to the multiple sensed signals fed back by the pressure sensor.

When the sensor is an optical sensor, the optical sensor can be arranged at a preset target position of the infusion pipe 304 between the infusion bag 40 and the extrusion mechanism 113 to output a corresponding feedback signal according to the information of liquid level in the infusion pipe 304 at the preset target position.

In this way, the infusion pump 100 can also determine the state information of the liquid level in the infusion apparatus according to the sensed signal fed back by the optical sensor.

In this embodiment, the control platform 102 can receive multiple sensed signals which are fed back by the pressure sensor according to a filtering period and filter the received sensed signals to obtain multiple sensed signals based on the filtering period. The control platform 102 can determine the trend of change in pressure on the pipe wall of the infusion apparatus 30 according to the multiple sensed signals based on the filtering period. In one embodiment, the control platform 102 can average the multiple sensed signals received in one filtering period to obtain an average value of the sensed signals corresponding to one filtering period, which is conducive to reducing the influence of the surrounding environment and patient activities on the sensed signals which are fed back by the pressure sensor. Among them, the sensed signal FiltPressAD corresponding to one filtering period can be expressed as:

$$FiltPressAD = \frac{1}{N}\sum_{i=k+1}^{k+N} PressAD_i;$$

Wherein, N refers to a number of sampling points in one filtering period, $PressAD_i$ refers to a sensed signal which is fed back by the pressure sensor at each sampling point, K refers to zero or a non-zero positive integer.

In one embodiment, when determining the filtering period, the control platform 102 determines the filtering period according to a preset fixed period, that is, N in the above formula refers to a fixed value. Alternatively, the control platform 102 determines the filtering period according to a current rotation period of the drive mechanism 113 of the infusion pump 100, that is, N in the above formula changes with the preset rotation period. The sensed signals of the sampling points in the corresponding filtering period can be selected by sliding a non-zero positive integer K. Alternatively, the control platform 102 determines the filtering period according to a current flow rate of the infusion pump 100, that is N in the above formula changes with a preset flow rate. For example, when the flow rate is 1ml/hr (millilitre per hour), N refers to 1000 sampling points, and when the flow rate is 100ml/hr, N refers to 100 sampling points.

In step S102, state information of a liquid level in the infusion apparatus 30 is determined according to the sensed signal.

Now refer FIG.5 simultaneously, which is a schematic diagram showing a relationship between a pressure signal transmitted by a pressure sensor during infusion and an infusion time, in an embodiment of the present disclosure. The dropper 302 is connected with the infusion bag 40 through the infusion pipe 304 with a preset length. Since the cross section of the infusion bag 40 is generally much larger than that of the infusion pipe 304, the liquid level in the infusion bag 40 does not fall into the infusion pipe 304 during a time duration of infusion (such as the time duration which is before time point Ta). At this time, the pressure sensor detects that the pressure signal on the pipe wall of the infusion pipe 304 is gradually reduced, and the pressure difference corresponding to the adjacent time intervals is relatively small. As shown in FIG. 5, the slope of the curve is relatively gently in a time duration before time point Ta. When the infusion is continued, as the liquid in the infusion bag 40 continues decreasing, the liquid level in the infusion bag 40 continues declining and then falls into the infusion pipe 304. At this time, the relationship corresponds to the curve after the time point Ta. Because the cross section of the infusion pipe 304 is small, the pressure information on the pipe wall which is fed back by the pressure sensor will gradually decrease at this time, and the pressure difference corresponding to the adjacent time intervals is greater than the pressure difference corresponding to the adjacent time intervals before the time point Ta. As shown in FIG. 5, the slope of the curve immediately after time point Ta is greater than that before the time point Ta. When the infusion is continued, the liquid level in the infusion pipe 304 will fall into the dropper 302. For example, at time point Tb, the liquid level in the infusion apparatus 30 falls into the dropper 302. Since the cross section of the dropper 302 is generally larger than that of the infusion pipe 304, the pressure difference corresponding to the adjacent time intervals after time point Tb is smaller than the pressure difference corresponding to the adjacent time intervals between time point Ta and time point Tb. As shown in FIG. 5, the slope of the curve after time point Tb is much gently than that between time point Ta and time point Tb.

Therefore, the control platform 102 of the infusion pump 100 can determine the state information of the liquid level in the infusion apparatus 30 according to the sensed signal which is fed back by the pressure sensor. For example, when the liquid level in the infusion bag 40 falls to the infusion apparatus 30, it indicates that the infusion bag 40 is empty.

In this embodiment, the infusion pump 100 can determine the trend of change in pressure on the pipe wall of the infusion apparatus 30 according to multiple sensed signals or the sensed signals which are filtered, and determine the state information of the liquid level in the infusion apparatus 30 according to the trend of change in pressure on the pipe wall.

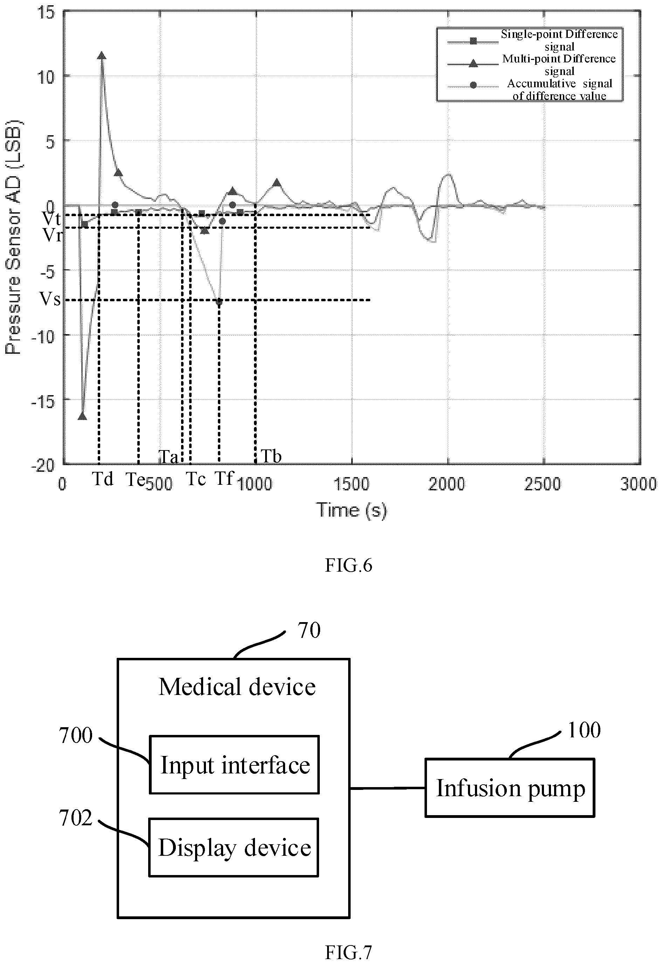

Now refer FIG.6 simultaneously, which is a schematic diagram showing a trend of change in pressure on a pipe wall under different modes, in an embodiment of the present disclosure.

The infusion pump 100 can determine the trend of change in pressure on the pipe wall of the infusion apparatus 30 according to a single-point difference method, a multi-point difference method or a difference accumulation method. Further, the infusion pump 100 can determine the time point Ta (i.e., the corresponding time point when the liquid level in the infusion bag 40 falls to the infusion apparatus 30) according to the trend of change in pressure on the pipe wall, so as to determine the state information of the liquid level in the infusion apparatus 30

The infusion pump 100 can determine difference values between multiple groups of the sensed signals according to the multiple sensed signals, wherein the difference values are difference values between the sensed signals at adjacent sampling time points (single-point difference method), or difference values between the sensed signals at sampling time points which are separated by a preset number of sampling time points (multi-point difference method).The trend of change in pressure on the pipe wall of the infusion apparatus 30 can be determined according to the difference values between the multiple groups of the sensed signals.

For example, the corresponding sensed signals of multiple sampling time points, are V1, V2, V3, V4 and V5 respectively. According to the single-point difference method, the difference values between the multiple groups of the sensed signals can include V2-V1, V3-V2, V4-V3 and V5-V4. According to the multiple-point difference method, the difference values between the multiple groups of the sensed signals can include V3-V1, V4-V2 and V5-V3. As shown in FIG.6, since the sensed signal gradually decreases with the time of the infusion, the difference value of the sensed signals in each group will be less than 0 in the single-point difference method. However, the difference value of the sensed signals in each group can be greater than 0 or less than 0 in the multi-point difference method.

In one embodiment, the infusion pump 100 can estimate the time point Ta when the liquid level in the infusion bag 40 enters into the infusion apparatus 30. For example, the infusion pump 100 can determine whether there is a target difference value of the sensed signals whose absolute value is greater than a first preset threshold (such as Vt) among the difference values between the multiple groups of the sensed signals. When there is a target difference value of the sensed signals whose absolute value is greater than the first preset threshold among the difference values between the multiple groups of the sensed signals, the infusion pump 100 can estimate that the liquid level in the infusion bag 40 enters into the infusion apparatus 30 at this time point (such as Tc). Further, the infusion pump 100 can determine that the state information of the liquid level in the infusion apparatus 30 has been determined at the time point Tc.

In one embodiment, when the target difference value of the sensed signals of the first preset threshold Vt is determined from the difference values between the multiple groups of the sensed signals, the infusion pump 100 obtains a target sampling time point (such as Tc) corresponding to the target difference value of the sensed signals. After that, if the infusion continues, the liquid level in the infusion apparatus 30 will continue falling, and the sensed signal which is fed back by the pressure sensor will continue decreasing. In order to determine the information of liquid level in the infusion bag 40 more accurately, the infusion pump 100 can continue obtaining a sensed comparison signal which is fed back by the pressure sensor after the target sampling time point, and determine whether a difference value between a sensed target signal and the sensed comparison signal is greater than a second preset threshold. When the difference value between the sensed target signal and the sensed comparison signal is greater than the second preset threshold, the state information of the liquid level in the infusion apparatus 30 is determined.

Please refer to FIG.5 again, at the beginning of infusion, the degree of change of the sensed signal which is fed back by the pressure sensor is relatively large, that is, the slope of the curve is steep. For the single-point difference method, for example, at the time point Td in FIG. 6, there may also be a target difference value of the sensed signals whose absolute value is greater than the first preset threshold (such as Vt) and there is still much liquid in the infusion bag 40 at this time. In order to improve the determination accuracy of the state information of the liquid level in the infusion apparatus 30, the infusion pump 100 can determine the time point Ta according to the single-point difference method after a preset time point (such as time point Te).

In one embodiment, the infusion apparatus 30 is vulnerable to the influence of the surrounding environment and the activities of patient, so certain interference is resulted in the sensed signal which is fed back by the pressure sensor. At this time, the infusion pump 100 can also adopt the multi-point difference method to improve the determination accuracy of the state information of the liquid level in the infusion apparatus 30. For example, refer to FIG.6 again, at the beginning of infusion, there is much liquid in the infusion bag 40 at this time, and the difference value of the sensed signals changes a lot before the time point Te when employing the multi-point difference method. Therefore, the infusion pump 100 can determine the difference value of the sensed signals after excluding the time points when the difference value of the sensed signals changes greatly. For example, the time point Ta can be determined according to the multiple-point difference method after the time point Te. The specific determination method is similar as that of the single-point difference method, so it will not be repeated here. The first preset threshold based on the single-point difference method can be different from the first preset threshold based on the multi-point difference method.

In one embodiment, since there is a certain length of infusion pipe 304 between the dropper 302 and the infusion bag 40, in order to improve the determination accuracy of the state information of the liquid level in the infusion apparatus 30, the infusion pump 100 can determine the state information of the liquid level in the infusion apparatus 30 after determining an accumulative pressure value within a preset time length after the time point Ta or an accumulative pressure value of the differential value of the multiple groups of sensed signals after the time point Ta.

Please refer to FIG.6 again, after determining the target sampling time point Ta corresponding to the target difference value of the sensed signals, the infusion pump 100 can accumulate the differential values of the multiple groups of the sensed signals after the target sampling time point as an accumulative pressure value, and determine whether an absolute value of the accumulative pressure value is greater than a third preset threshold (such as, absolute value Vs). When the accumulative pressure value is greater than the third preset threshold, the infusion pump 100 can determine the state information of the liquid level in the infusion apparatus 30. For example, when the infusion pump 100 determines that the absolute value of the accumulative pressure value is greater than the third preset threshold at a time point Tf, the infusion pump 100 can determine the state information of the liquid level in the infusion apparatus 30 at the time point Tf.

The infusion pump 100 can accumulate a number of the sampling time points after the target sampling time point as an accumulative sampling frequency (such as the preset time length), and can determine whether the accumulative sampling frequency is greater than a fourth preset threshold. When the accumulative sampling frequency is greater than the fourth preset threshold, the infusion pump 100 determines the state information of the liquid level in the infusion apparatus 30.

In one embodiment, the sensor may be an optical sensor, wherein the optical sensor may be arranged on the infusion pipe 304 between the infusion bag 40 and the extrusion mechanism 113. The optical sensor is operable to feed back an optical signal, wherein the optical signal characterizes a gas state within a preset target position of the infusion apparatus 30. Further, the infusion pump 100 can determine that the liquid level in the infusion apparatus 30 reaches the preset target position according to the optical signal.

In one embodiment, the infusion pump 100 may determine an amount of gas which passes through or passing time of gas which exceeds a preset bubble threshold, at the preset target position in the infusion apparatus 30, according to the optical signal which is fed back by the optical sensor.

The infusion pump 100 can determine that the liquid level of the infusion apparatus 30 reaches the preset target position according to the amount of gas which passes through the preset target position and a preset gas accumulative value. For example, when the amount of gas which passes through exceeds the preset gas accumulative value, the infusion pump 100 can determine the state information of the liquid level in the infusion apparatus 30.

The infusion pump 100 can also determine that the liquid level of the infusion apparatus 30 reaches the preset target position according to the passing time of gas at the preset target position and a preset gas accumulative time. For example, when the passing time of gas reaches the preset gas accumulative time, the infusion pump 100 can determine the state information of the liquid level in the infusion apparatus 30.

In step S104, implementation of at least one of following events is triggered according to the state information of the liquid level:

> outputting alarm information,
> driving an extrusion mechanism of the infusion pump to stop moving, or
> driving the extrusion mechanism of the infusion pump to slow down so as to adjust an infusion flow rate of the infusion pump.

In this embodiment, when the infusion pump 100 determines the state information of the liquid level of the infusion apparatus 30, the infusion pump 100 can output alarm information, which includes but is not limited to one or more of sound, light, text or chart. For example, the control platform 102 may output text or chart information through the display system 160 or control the light component 168 to output alarm information of light through the light controller 148, such as alarm information for controlling the color or brightness of the light to change with the tone and frequency of the alarm sound.

In this embodiment, after the infusion pump 100 determines the state information of the liquid level, the infusion pump 100 can control the extrusion mechanism 113 to stop moving or to slow down so as to adjust a flow rate of infusion of the infusion pump 100, thus reducing the probability of medical accidents. In one embodiment, the infusion pump 100 is also operable to drive the liquid stopping clip to open, when determining that at least a part of the infusion apparatus 30 is separated from the infusion pipeline 115 according to the position sensor after the extrusion mechanism 113 of the infusion pump 100 stops moving.

The above infusion state detection method for an infusion pump determines the state information of the liquid level in the infusion apparatus according to the sensed signal which is fed back by the sensor, and outputs the alarm information or controls the movement state of the extrusion mechanism when the state information of the liquid level in the infusion apparatus is determined. In this way, when the liquid in the infusion bag enters into the infusion apparatus, an instruction can be sent timely, or the movement of the extrusion mechanism can be controlled timely. This is beneficial to reduce the additional operation of the medical staff and reduce the probability of medical accidents.

Please refer to FIG.7, which is a schematic diagram showing a connection between a medical device and an infusion pump, in an embodiment of the present disclosure. In this embodiment, a medical device 70 is connected with the infusion pump 100 in a detachable fixation manner, wherein an input interface 700 of the medical device 70 is connected with the output interface of the infusion pump 100. The medical device 70 further includes a display device 702. When determining the state information of the liquid level of the infusion apparatus 30, the infusion pump 100 outputs the alarm information to the medical device 70 to display the alarm information through the display device 702 of the medical device 70. In this embodiment, the medical device 70 may also include a processor, a memory, etc. The processor of the medical device 70 may control the display of the received alarm information through the display device 702.

In the above embodiments, the description of each embodiment has its own emphasis. For the parts not detailed in one embodiment, please refer to the relevant description of other embodiments.

In several embodiments provided in the present application, it should be understood that the disclosed devices can be realized in other ways. For example, the device embodiments described above are only schematic, for example, the division of the components is only a logical function division, and there can be another division mode in actual implementation, for example, multiple units or components can be combined or integrated into another system, or some features can be ignored or not executed. On the other hand, the mutual coupling or direct coupling or communication connection shown or discussed can be indirect coupling or communication connection through some interfaces, devices or units, and can be electrical or in other forms.

The unit described as a separate component may or may not be physically separated, and the component displayed as a unit may or may not be a physical unit, that is, it may be located in one place or distributed to multiple network units. Some or all of the units can be selected according to the actual needs to achieve the purpose of the embodiment.

In addition, each functional unit in each embodiment of the application can be integrated into one processing unit. Each unit can exist separately, or two or more units can be integrated into one unit. The above integrated units can be realized in the form of hardware or software functional units.

The embodiments of this disclosure have been introduced in detail above. In this disclosure, specific examples have been used to explain the principle and implementation mode of this disclosure. The description of the above embodiments is only used to help those skilled in the art to understand the method and core idea of this disclosure. Meanwhile, for those skilled in the art, there will be changes in the specific implementation mode and application scope according to the idea of this disclosure. In conclusion, the contents of the specification should not be understood as restrictions on this disclosure.

## Claims

1. An infusion state detection method for an infusion pump, **characterized in that**, the method is applied to an infusion pump system, wherein the infusion pump system comprises an infusion pump and an infusion apparatus which is arranged along an infusion pipeline; wherein the method comprises:

acquiring a sensed signal which is fed back by a sensor, wherein the sensor is arranged on the infusion apparatus;
determining state information of a liquid level in the infusion apparatus according to the sensed signal;
triggering implementation of at least one of following events according to the state information of the liquid level:

outputting alarm information,
driving an extrusion mechanism of the infusion pump to stop moving, or
driving the extrusion mechanism of the infusion pump to slow down so as to adjust an infusion flow rate of the infusion pump.

2. The infusion state detection method for an infusion pump according to claim 1, **characterized in that**, the sensor is arranged inside the infusion pump, or the sensor is arranged inside a housing which is arranged independently of the infusion pump.

3. The infusion state detection method for an infusion pump according to claim 2, **characterized in that**, the infusion apparatus includes a dropper and the sensor is a pressure sensor which is arranged between the dropper and the extrusion mechanism of the infusion pump;
wherein acquiring a sensed signal which is fed back by a sensor and determining state information of a liquid level in the infusion apparatus according to the sensed signal, comprise:

acquiring multiple sensed signals which are fed back by the pressure sensor, wherein the multiple sensed signals characterize pressure information on a pipe wall of the infusion apparatus, and the pipe wall of the infusion apparatus is between the infusion apparatus and the extrusion mechanism of the infusion pump;
determining a trend of change in pressure on the pipe wall of the infusion apparatus according to the multiple sensed signals; and
determining the state information of the liquid level in the infusion apparatus according to the trend of change in pressure on the pipe wall.

4. The infusion state detection method for an infusion pump according to claim 3, **characterized in that**, determining a trend of change in pressure on the pipe wall of the infusion apparatus according to the multiple sensed signals, comprises:

determining difference values between multiple groups of the sensed signals according to the multiple sensed signals, wherein the difference values are difference values between the sensed signals at adjacent sampling time points, or difference values between the sensed signals at sampling time points which are separated by a preset number of sampling time points; and determining the trend of change in pressure on the pipe wall of the infusion apparatus according to the difference values between multiple groups of the sensed signals.

**5.** The infusion state detection method for an infusion pump according to claim 4, **characterized in that**, determining the state information of the liquid level in the infusion apparatus according to the trend of change in pressure on the pipe wall, comprises:

determining whether there is a target difference value of the sensed signals whose absolute value is greater than a first preset threshold, among the difference values between multiple groups of the sensed signals;
acquiring a target sampling time point corresponding to the target difference value of the sensed signals, when there is a target difference value of the sensed signals whose absolute value is greater than the first preset threshold;
acquiring a sensed comparison signal after the target sampling time point;
determining whether a difference value between a sensed target signal and the sensed comparison signal is greater than a second preset threshold; and
determining the state information of the liquid level in the infusion apparatus when the difference value between the sensed target signal and the sensed comparison signal is greater than the second preset threshold.

**6.** The infusion state detection method for an infusion pump according to claim 5, **characterized in that**, after acquiring a target sampling time point corresponding to the target difference value of the sensed signals, the method further comprises:

accumulating difference values between multiple groups of the sensed signals after the target sampling time point as an accumulative pressure value,
determining whether an absolute value of the accumulative pressure value is greater than a third preset threshold, and
determining the state information of the liquid level in the infusion apparatus when the absolute value of the accumulative pressure value is greater than the third preset threshold; or accumulating a number of the sampling time points after the target sampling time point as an accumulative sampling frequency,
determining whether the accumulative sampling frequency is greater than a fourth preset threshold, and
determining the state information of the liquid level in the infusion apparatus when the accumulative sampling frequency is greater than the fourth preset threshold.

**7.** The infusion state detection method for an infusion pump according to claim 3, **characterized in that**: acquiring multiple sensed signals which are fed back by the pressure sensor, comprises:

determining a filtering period, and filtering the multiple sensed signals in the filtering period to obtain multiple sensed signals which are based on the filtering period;
determining a trend of change in pressure on the pipe wall of the infusion apparatus according to the multiple sensed signals, comprises:
determining the trend of change in pressure on the pipe wall of the infusion apparatus according to the multiple sensed signals which are based on the filtering period.

**8.** The infusion state detection method for an infusion pump according to claim 7, **characterized in that**, determining a filtering period, comprises:

determining the filtering period according to a preset fixed period; or
determining the filtering period according to a current rotation period of a drive mechanism of the infusion pump; or
determining the filtering period according to a current flow rate of the infusion pump.

**9.** The infusion state detection method for an infusion pump according to claim 1, **characterized in that**, the sensor is an optical sensor which is arranged between an infusion bag and the extrusion mechanism; wherein acquiring a sensed signal which is fed back by a sensor and determining state information of the liquid level in the infusion apparatus according to the sensed signal, comprise:

acquiring an optical signal which is fed back by the optical sensor, wherein the optical signal characterizes a gas state within a preset target position of the infusion apparatus;
determining that the liquid level in the infusion apparatus reaches the preset target position according to the optical signal.

**10.** The infusion state detection method for an infusion pump according to claim 9, **characterized in that**, determining that the liquid level in the infusion apparatus reaches a preset target position according to the optical signal, comprises:

determining an amount of gas which passes through the preset target position in the infusion apparatus or passing time of gas which exceeds a preset bubble threshold at the preset target position in the infusion apparatus, according to the optical signal which is fed back by the optical sensor; and

determining that the liquid level of the infusion apparatus reaches the preset target position according to the amount of gas which passes through and a preset gas accumulative value, or determining that the liquid level of the infusion apparatus reaches the preset target position according to the passing time of gas and a preset gas accumulative time.

**11.** An infusion pump, **characterized in that**, the infusion pump is used together with an infusion apparatus and operable to implement an infusion and injection operation on a liquid which is prepared by a user; wherein the infusion pump comprises a processor, an output interface, a sensor, a drive mechanism and an extrusion mechanism; wherein the processor is operable to drive the drive mechanism to enable the extrusion mechanism to squeeze the liquid in the infusion apparatus for moving the liquid according to a preset direction;

the infusion apparatus is arranged along an infusion pipeline, the sensor is arranged on the infusion apparatus;

wherein the sensor is operable to feed back to the processor a sensed signal which is associated with a state of the infusion apparatus; and the processor is further operable to acquire the sensed signal, which is fed back by the sensor, to determine state information of a liquid level in the infusion apparatus according to the sensed signal, and to trigger implementation of at least one of following events according to the state information of the liquid level:

outputting alarm information through the output interface,

driving the extrusion mechanism of the infusion pump to stop moving, or driving the extrusion mechanism of the infusion pump to slow down so as to adjust a flow rate of the infusion pump.

**12.** The infusion pump according to claim 11, **characterized in that**, the sensor is arranged inside the infusion pump, or the sensor is arranged inside a housing which is arranged independently of the infusion pump.

**13.** The infusion pump according to claim 12, **characterized in that**, the infusion apparatus includes a dropper, and the sensor is a pressure sensor which is arranged between the dropper and the extrusion mechanism of the infusion pump; wherein when the processor is operable to acquire the sensed signal, which is fed back by the sensor, to determine state information of a liquid level in the infusion apparatus according to the sensed signal, the processor is specifically operable to:

acquire multiple sensed signals which are fed back by the pressure sensor, wherein the multiple sensed signals characterize pressure information on a pipe wall of the infusion apparatus, and the pipe wall of the infusion apparatus is between the infusion apparatus and the extrusion mechanism of the infusion pump, determine a trend of change in pressure on the pipe wall of the infusion apparatus according to the multiple sensed signals, and determine the state information of the liquid level in the infusion apparatus according to the trend of change in pressure on the pipe wall.

**14.** The infusion pump according to claim 13, **characterized in that**, when the processor is operable to determine a trend of change in pressure on the pipe wall of the infusion apparatus according to the multiple sensed signals, the processor is specifically operable to:

determine difference values between multiple groups of the sensed signals according to the multiple sensed signals, wherein the difference values are difference values between the sensed signals at adjacent sampling time points, or difference values between the sensed signals at sampling time points which are separated by a preset number of sampling time points; and determine the trend of change in pressure on the pipe wall of the infusion apparatus according to the difference values between multiple groups of the sensed signals.

**15.** The infusion pump according to claim 14, **characterized in that**, when the processor is operable to determine the state information of a liquid level in the infusion apparatus according to the trend of change in pressure on the pipe wall, the processor is specifically operable to:

determine whether there is a target difference value of the sensed signals whose absolute value is greater than a first preset threshold, among the difference values between multiple groups of the sensed signals;

acquire a target sampling time point corresponding to the target difference value of the sensed signals, when there is a target difference value of the sensed signals whose absolute value is greater than the first preset threshold;

acquire a sensed comparison signal after the target sampling time point;

determine whether a difference value between a sensed target signal and the sensed comparison signal is greater than a second preset threshold; and

determine the state information of the liquid level in the infusion apparatus when the difference value between the sensed target signal and the sensed comparison signal is greater than the second preset threshold.

16. The infusion pump according to claim 15, **characterized in that**, after the processor is operable to acquire a target sampling time point corresponding to the target difference value of the sensed signals, the processor is further operable to:

accumulate difference values between multiple groups of the sensed signals after the target sampling time point as an accumulative pressure value,

determine whether an absolute value of the accumulative pressure value is greater than a third preset threshold, and

determine the state information of the liquid level in the infusion apparatus when the absolute value of the accumulative pressure value is greater than the third preset threshold; or

accumulate a number of the sampling time points after the target sampling time point as an accumulative sampling frequency,

determine whether the accumulative sampling frequency is greater than a fourth preset threshold, and

determine the state information of the liquid level in the infusion apparatus when the accumulative sampling frequency is greater than the fourth preset threshold.

17. The infusion pump according to claim 12, **characterized in that**, after the processor is operable to acquire multiple sensed signals which are fed back by the pressure sensor, the processor is further operable to determine a filtering period, and filter the multiple sensed signals in the filtering period to obtain multiple sensed signals which are based on the filtering period;

when the processor is operable to determine a trend of change in pressure on the pipe wall of the infusion apparatus according to the multiple sensed signals, the processor is specifically operable to determine the trend of change in pressure on the pipe wall of the infusion apparatus according to the multiple sensed signals which are based on the filtering period.

18. The infusion pump according to claim 11, **characterized in that**, the sensor is an optical sensor which is arranged between an infusion bag and the extrusion mechanism; wherein the processor is operable to acquire an optical signal which is fed back by the optical sensor, wherein the optical signal characterizes a gas state within a preset target position of the infusion apparatus;

wherein the processor is further operable to determine that the liquid level in the infusion apparatus reaches the preset target position according to the optical signal.

19. The infusion pump according to claim 18, **characterized in that**, the processor is specifically operable to:

determine an amount of gas which passes through the preset target position in the infusion apparatus or passing time of gas which exceeds a preset bubble threshold at the preset target position in the infusion apparatus, according to the optical signal which is fed back by the optical sensor; and

determine that the liquid level of the infusion apparatus reaches the preset target position according to the amount of gas which passes through and a preset gas accumulative value, or determine that the liquid level of the infusion apparatus reaches the preset target position according to the passing time of gas and a preset gas accumulative time.

20. An infusion pump, **characterized in that**, the infusion pump is used together with an infusion apparatus and is operable to implement an infusion and injection operation on a liquid which is prepared by a user; wherein the infusion pump comprises a processor, an output interface, a sensor, a drive mechanism, an extrusion mechanism, a position sensor and a liquid stopping clip, wherein the infusion apparatus is arranged along an infusion pipeline and the sensor is arranged on the infusion pipeline;

wherein the sensor is operable to feed back to the processor a sensed signal which is associated with a state of the infusion apparatus, the position sensor is operable to detect an arrangement state of the infusion apparatus on the infusion pipeline and to send the arrangement state of the infusion apparatus to the processor;

wherein the processor is operable to drive the drive mechanism to enable the extrusion mechanism to squeeze the liquid in the infusion apparatus for moving the liquid according to a preset direction, to acquire the sensed signal which is fed back by the sensor, to determine state information of a liquid level in the infusion apparatus according to the sensed signal, and to trigger implementation of at least one of following events according to the state information of the liquid level:

outputting alarm information through the output interface,
driving the extrusion mechanism of the infusion pump to stop moving, or
driving the extrusion mechanism of the infusion pump to slow down so as to adjust a flow rate of the infusion pump;
wherein the processor is further operable to drive the liquid stopping clip to open, when determining that at least a part of the infusion apparatus is separated from the infusion pipeline according to the arrangement state of the infusion apparatus after the extrusion mechanism of the infusion pump stops moving.

21. A medical device, **characterized in that**, the medical device is connected with the infusion pump according to any one of claims 11-20 in a detachable fixation manner, wherein an input interface of the medical device is connected with the output interface of the infusion pump, and a display device is arranged on the medical device to display the alarm information outputted by the output interface.

22. A computer-readable storage medium on which executable instructions are stored, wherein when the executable instructions are executed by a processor, the infusion state detection method for an infusion pump according to any one of claims 1-10 is implemented.

```
                                                            ⌐100
┌─────────────────────────────────────────────────────────────┐
│      Acquiring a sensed signal which is fed back by a sensor, │
│      wherein the sensor is arranged on an infusion apparatus  │
└─────────────────────────────────────────────────────────────┘
                              │
                              ▼                             ⌐102
┌─────────────────────────────────────────────────────────────┐
│      Determining state information of liquid level in the     │
│      infusion apparatus according to the sensed signal        │
└─────────────────────────────────────────────────────────────┘
                              │
                              ▼                             ⌐104
┌─────────────────────────────────────────────────────────────┐
│      Triggering an implementation of at least one of following│
│      events according to the state information of liquid level:│
│      outputting alarm information, driving an extrusion mechanism│
│      of the infusion pump to stop moving, or driving the extrusion│
│      mechanism of the infusion pump to slow down so as to adjust│
│      an infusion flow rate of the infusion pump               │
└─────────────────────────────────────────────────────────────┘
```

FIG.1

```
                                          ↙ 50
            ⌐ 100
┌──────────────────────┐          ⌐ 30
│    Infusion pump      │   ┌──────────────────────────┐
│                       │   │    Infusion apparatus     │
│  ┌─────────────────┐  │   │                           │
│115│    Infusion     │  │   │                       ⌐302│
│  │    pipeline      │──┼───│  ┌─────────────────┐     │
│  └─────────────────┘  │   │  │    Dropper       │     │
│  ┌─────────────────┐  │   │  └─────────────────┘     │
│117│   Pump door     │  │   │                       ⌐304│
│  └─────────────────┘  │   │  ┌─────────────────┐     │
│  ┌─────────────────┐  │   │  │  Infusion pipe   │     │
│119│   Pump body     │  │   │  └─────────────────┘     │
│  └─────────────────┘  │   └──────────────────────────┘
└──────────────────────┘
```

FIG.2

10

102

101

Peripheral device interface 152 ⟷ Processor/ controller 150

Power system 106

101

Power drive circuit 130

RF circuit 120

156

104

Operation system 170

Communication module 172

Drop sensor 132

External interface 122

Touch module 174

Tactile feedback module 176

154

Bubble sensor 134

Audio circuit 124

Movement module 178

Position module 180

Pressure sensor 136

Monitoring circuit 126

Pattern module 182

Text input module 190

Temperature sensor 138

Protection circuit 128

Device/overall internal state 192

Application 194

Optical sensor 139

Infusion mode setting 194-1

Blocking pressure level setting 194-2

I/O system 108

Display system 160

Display controller 140

Bubble level setting 194-3

Drug setting 194-4

Other input/control devices 162

Other input controller 142

Volume setting 194-5

Brightness setting 194-6

Position sensor 164

Position sensor controller 144

Online setting 195-7

Displacement sensor 166

Proximity sensor controller 146

Dock setting 195-8

Temperature setting 195-9

168

⊗

Light controller 148

......

FIG.3

FIG.4

FIG.5

FIG.6

FIG.7

## INTERNATIONAL SEARCH REPORT

| International application No. |
|---|
| **PCT/CN2019/096009** |

**A. CLASSIFICATION OF SUBJECT MATTER**

A61M 5/172(2006.01)i

According to International Patent Classification (IPC) or to both national classification and IPC

**B. FIELDS SEARCHED**

Minimum documentation searched (classification system followed by classification symbols)

A61M 5/-

Documentation searched other than minimum documentation to the extent that such documents are included in the fields searched

Electronic data base consulted during the international search (name of data base and, where practicable, search terms used)

WPI, EPODOC, CNABS, CNTXT, CNKI: 深圳迈瑞科技有限公司, 左鹏飞, 涂有强, 输液, 传感器, 压力, 挤, 液面, 液位, 差分, 压力变化, 流速, 报警, 停止, 关闭, 减慢, 减缓, 滤波周期, infusion pump, liquid level, differential value, filter+, period, pressure sensor, chang+, alarm+

**C. DOCUMENTS CONSIDERED TO BE RELEVANT**

| Category* | Citation of document, with indication, where appropriate, of the relevant passages | Relevant to claim No. |
|---|---|---|
| X | CN 104582759 A (TERUMO CORPORATION) 29 April 2015 (2015-04-29) description, paragraphs [0043]-[0121], figures 1, 3-4, 11 | 1-2, 9-12, 18-22 |
| Y | CN 104582759 A (TERUMO CORPORATION) 29 April 2015 (2015-04-29) description, paragraphs [0043]-[0121], figures 1, 3-4, 11 | 1-4, 9-14, 18-22 |
| Y | CN 106362234 A (JI, Bing et al.) 01 February 2017 (2017-02-01) description, paragraph [0059] | 1-4, 9-14, 18-22 |
| Y | CN 109731182 A (GOERTEK TECHNOLOGY CO., LTD.) 10 May 2019 (2019-05-10) description, paragraphs [0024], [0033] | 3-4, 13-14 |
| A | CN 109414537 A (FRESENIUS MEDICAL CARE HOLDINGS INC) 01 March 2019 (2019-03-01) entire document | 1-22 |
| A | CN 102058914 A (254 HOSPITAL OF PLA) 18 May 2011 (2011-05-18) entire document | 1-22 |
| A | JP 09164201 A (OMRON TATEISI ELECTRONICS CO.) 24 June 1997 (1997-06-24) entire document | 1-22 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 March 2020** | **13 April 2020** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/096009**

| Patent document cited in search report | | | Publication date (day/month/year) | Patent family member(s) | | | Publication date (day/month/year) |
|---|---|---|---|---|---|---|---|
| CN | 104582759 | A | 29 April 2015 | JP | WO2014118944 | A1 | 26 January 2017 |
| | | | | JP | 6240620 | B2 | 29 November 2017 |
| | | | | CN | 104582759 | B | 19 January 2018 |
| | | | | WO | 2014118944 | A1 | 07 August 2014 |
| CN | 106362234 | A | 01 February 2017 | CN | 106362234 | B | 12 November 2019 |
| CN | 109731182 | A | 10 May 2019 | | None | | |
| CN | 109414537 | A | 01 March 2019 | AU | 2017288251 | A1 | 08 November 2018 |
| | | | | US | 2018001009 | A1 | 04 January 2018 |
| | | | | JP | 2019519331 | A | 11 July 2019 |
| | | | | CA | 3021353 | A1 | 04 January 2018 |
| | | | | EP | 3426318 | A1 | 16 January 2019 |
| | | | | WO | 2018005076 | A1 | 04 January 2018 |
| CN | 102058914 | A | 18 May 2011 | | None | | |
| JP | 09164201 | A | 24 June 1997 | JP | H09164201 | A | 24 June 1997 |

Form PCT/ISA/210 (patent family annex) (January 2015)